# EUROPEAN PATENT APPLICATION

(11) **EP 3 954 693 A1**
(43) Date of publication of application: **16.02.2022**
(21) Application number: 20788192.1
(22) Date of filing: 10.04.2020
(51) Int. Cl.: C07D 498/10, A61P 35/00, A61K 31/505

(54) **CRYSTAL FORM OF EGFR INHIBITOR AND PREPARATION METHOD THEREFOR**

(30) Priority: 10.04.2019 CN 201910285738
(71) Applicant: Medshine Discovery Inc., Nanjing, Jiangsu 210032 (CN)
(72) Inventor: LIU, Xile, Shanghai 200131 (CN); ZHANG, Lu, Shanghai 200131 (CN); DING, Charles Z., Shanghai 200131 (CN); CHEN, Shuhui, Shanghai 200131 (CN); HU, Lihong, Shanghai 200131 (CN)
(74) Representative: De Clercq & Partners
(86) International application number: PCT/CN2020/084286
(87) International publication number: WO 2020/207483

(57) **Abstract**

Disclosed are a crystal form of an EGFR inhibitor and a preparation method therefor. Further disclosed is an application of the crystal form in preparing a drug for treating non-small cell lung cancer, especially an application in preparing a drug for treating non-small cell lung cancer with brain metastases.

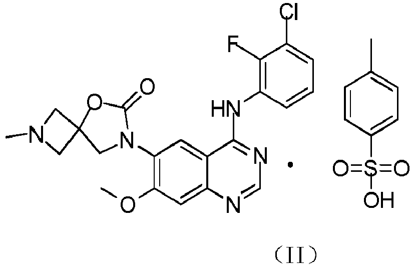

## Description

This application claims the priority of:
CN201910285738.X, filed on April 10, 2019.

### FIELD OF THE INVENTION

The present disclosure relates to a crystal form of an EGFR inhibitor and a preparation method thereof, and also includes the use of the crystal form in the manufacture of a medicament for treating non-small cell lung cancer, especially brain metastases in non-small cell lung cancer.

### BACKGROUND OF THE INVENTION

EGFR (epidermal growth factor receptor, abbreviated as EGFR, ErbB-1, or HER1) is a member of the epidermal growth factor receptor (HER) family. This family includes HER1 (erbB1, EGFR), HER2 (erbB2), HER3 (erbB3), and HER4 (erbB4). EGFR is a glycoprotein that is a receptor for epidermal growth factor (EGF) cell proliferation and signaling, which belongs to tyrosine kinase receptor. EGFR crosses cell membrane and is located on the surface of the cell membrane. After binding of ligand to epidermal growth factor receptor (EGFR), the receptor will undergo dimerization. The dimerization of EGFR can activate the kinase pathway of EGFR in cells. This autophosphorylation can guide downstream phosphorylation, including MAPK, Akt, and JNK pathways, thereby inducing cell proliferation.

However, drug resistance will appear after using EGFR-TKI for a certain period of time, and about one-third of patients develop CNS metastasis after acquiring EGFR-TKI resistance. NSCLC patients with brain metastases have poor quality of life and poor prognosis, and their natural median survival time is only 1-2 months. At present, fewer treatment methods are available for brain metastases, in which single and isolated lesions are mostly treated with surgery or stereotactic radiotherapy, while multiple lesions are mainly treated with whole-brain radiotherapy. Although whole brain radiotherapy can prolong the survival period of patients to a certain extent, the curative effect is still not ideal, and toxic side effects are large. Due to the existence of blood-brain barrier (BBB), it is difficult for many drugs to penetrate the blood-brain barrier and enter brain tissue, and the effective therapeutic concentration cannot be achieved in the brain. Therefore, the use of EGFR-TKI in these patients is not effective.

Although no molecular targeted therapy drugs specifically for brain metastases in NSCLC have been approved for marketing at present, a large number of clinical studies in recent years have shown that molecular targeted drugs provide new treatment options for brain metastases in NSCLC.

AZD3759 is a new type of targeted drug with the ability of entering the brain efficiently, which is developed by AstraZeneca and is in clinical phase I/II at present. AZD3759 is used to address central nervous system (CNS) metastases in patients with EGFR mutation-positive non-small cell lung cancer, such as brain metastases (BM) and leptomeningeal metastases (or Leptomeningeal Metastasis, LM). AZD3759 has good blood-brain barrier permeability. From the current clinical trials, AZD3759 has a high clinical response rate, and has significant efficacy in both extracranial and intracranial patients.

Erlotinib is approved by the FDA for initial (first-line) treatment of patients with metastatic non-small cell lung cancer (NSCLC) whose tumors have been confirmed to have specific epidermal growth factor receptor (EGFR) activating mutations by testing. Moreover, the drug has also been approved for the treatment of advanced NSCLC patients whose tumors have spread or grown after receiving at least one chemotherapy regimen (second-line or third-line treatment).

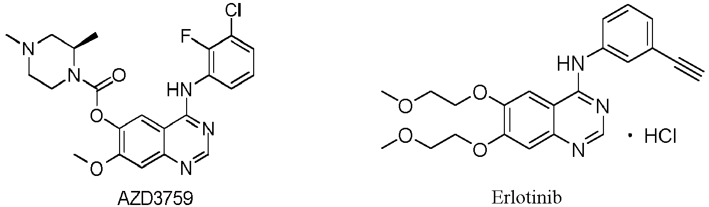

### SUMMARY OF THE INVENTION

The present disclosure provides a crystal form A of the compound of formula (II), which has an X-ray powder diffraction pattern having characteristic diffraction peaks at 2θ angles of: 6.04±0.2°, 12.07±0.2°, and 13.32±0.2°.

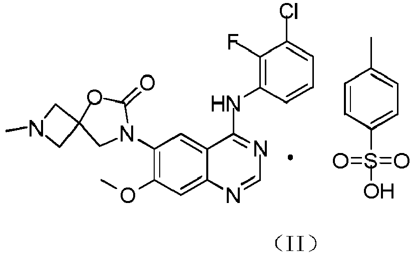

In some embodiments of the present disclosure, the X-ray powder diffraction pattern of the above-mentioned crystal form A has characteristic diffraction peaks at 2θ angles of: 6.04±0.2°, 12.07±0.2°, 13.32±0.2°, 16.06±0.2°, 19.01 ±0.2°, 20.14±0.2°, 25.07±0.2°, and 30.44±0.2° .

In some embodiments of the present disclosure, the X-ray powder diffraction pattern of the above-mentioned crystal form A has characteristic diffraction peaks at 2θ angles of: 6.04±0.2°, 8.26±0.2°, 10.79±0.2°, 11.27±0.2°, 12.07 ±0.2°, 13.32±0.2°, 16.06±0.2°, 19.01±0.2°, 20.14±0.2°, 23.02±0.2°, 25.07±0.2°, and 30.44±0.2°.

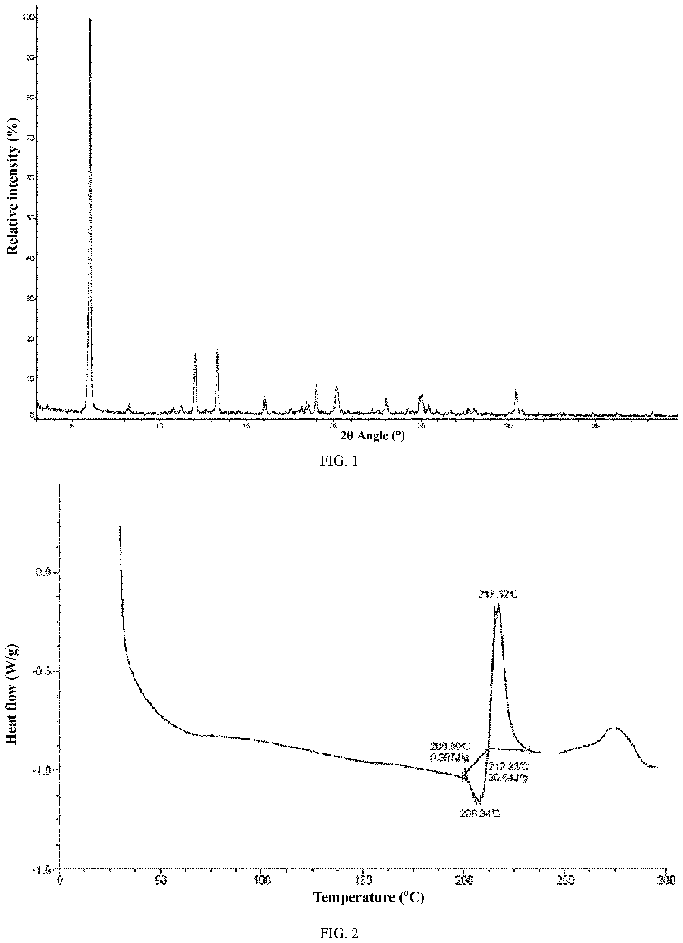

In some embodiments of the present disclosure, the XRPD pattern of the above-mentioned crystal form A is as shown in FIG. 1.

In some embodiments of the present disclosure, the analysis data of the XRPD pattern of the above-mentioned crystal form A is as shown in Table 1.

**Table 1 Analysis data of the XRPD pattern of the crystal form A of the compound (II)**

| **No.** | **2θ Angle (°)** | **D-spacing (Å)** | **Relative intensity (%)** | **No.** | **2θ Angle (°)** | **D-spacing (Å)** | **Relative intensity (%)** |
|---|---|---|---|---|---|---|---|
| 1 | 6.038 | 14.6258 | 100 | 14 | 19.316 | 4.5914 | 0.8 |
| 2 | 8.263 | 10.6918 | 3.1 | 15 | 20.140 | 4.4054 | 7.2 |
| 3 | 10.789 | 8.1934 | 2.0 | 16 | 22.171 | 4.0062 | 1.4 |
| 4 | 11.268 | 7.8462 | 2.0 | 17 | 22.512 | 3.9462 | 0.7 |
| 5 | 12.074 | 7.3242 | 15.1 | 18 | 23.021 | 3.8602 | 3.8 |
| 6 | 12.738 | 6.9439 | 0.9 | 19 | 24.252 | 3.6669 | 1.4 |
| 7 | 13.318 | 6.6424 | 16.1 | 20 | 25.067 | 3.5494 | 4.9 |
| 8 | 16.056 | 5.5155 | 4.6 | 21 | 25.443 | 3.4979 | 2.3 |
| 9 | 17.555 | 5.0478 | 1.3 | 22 | 26.629 | 3.3448 | 1.1 |
| 10 | 18.154 | 4.8826 | 1.9 | 23 | 27.699 | 3.2179 | 1.7 |
| 11 | 18.425 | 4.8115 | 2.9 | 24 | 28.053 | 3.1781 | 1.6 |
| 12 | 18.577 | 4.7723 | 2.1 | 25 | 30.440 | 2.9341 | 6.4 |
| 13 | 19.014 | 4.6635 | 7.2 | 26 | 30.787 | 2.9018 | 1.3 |

In some embodiments of the present disclosure, the above-mentioned crystal form A has a differential scanning calorimetry curve having an onset of an endothermic peak at 200.99±5°C and an onset of an exothermic peak at 212.33±5°C.

In some embodiments of the present disclosure, the DSC curve of the above-mentioned crystal form A is as shown in FIG. 2.

In some embodiments of the present disclosure, the above-mentioned crystal form A has a thermogravimetric analysis curve having a weight loss of up to 0.2289% at 120.00±3°C.

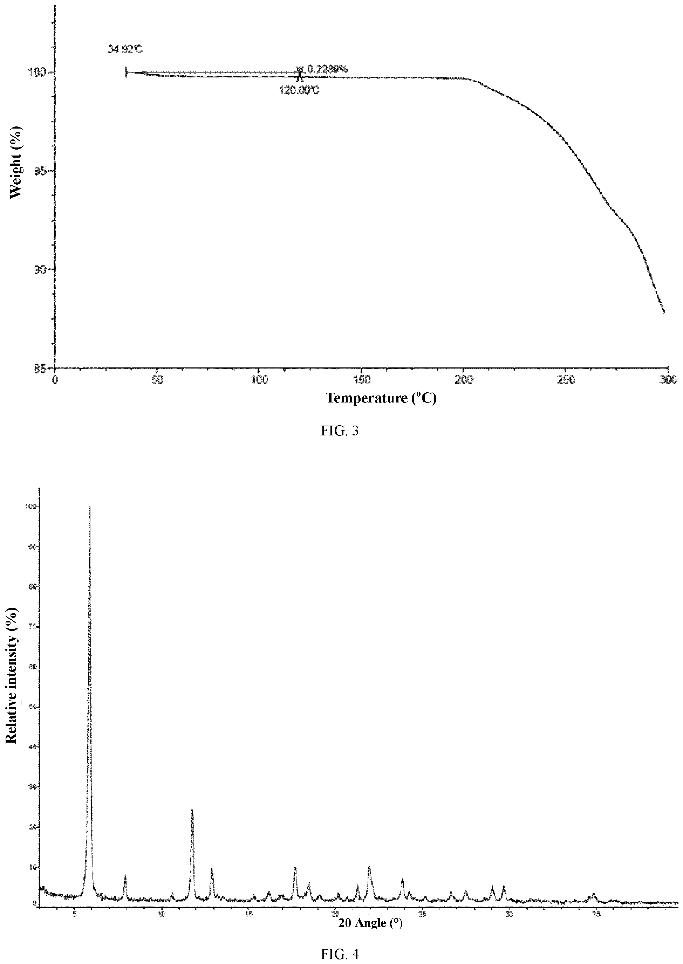

In some embodiments of the present disclosure, the TGA curve of the above-mentioned crystal form A is as shown in FIG. 3.

The present disclosure provides a crystal form B of the compound of formula (II), which has an X-ray powder diffraction pattern having characteristic diffraction peaks at 2θ angles of: 5.88±0.2°, 11.79±0.2°, and 21.96±0.2°.

In some embodiments of the present disclosure, the X-ray powder diffraction pattern of the above-mentioned crystal form B has characteristic diffraction peaks at 2θ angles of: 5.88±0.2°, 7.92±0.2°, 11.79±0.2°, 12.92±0.2°, 17.68 ±0.2°, 18.50±0.2°, 21.96±0.2°, and 23.87±0.2°.

In some embodiments of the present disclosure, the X-ray powder diffraction pattern of the above-mentioned crystal form B has characteristic diffraction peaks at 2θ angles of: 5.88±0.2°, 7.92±0.2°, 11.79±0.2°, 12.92±0.2°, 17.68 ±0.2°, 18.50±0.2°, 21.29±0.2°, 21.96±0.2°, 23.87±0.2°, 27.52±0.2°, 29.05±0.2°, and 29.69±0.2°.

In some embodiments of the present disclosure, the XRPD pattern of the above-mentioned crystal form B is as shown in FIG. 4.

In some embodiments of the present disclosure, the analysis data of the XRPD pattern of the above-mentioned crystal form B is as shown in Table 2.

**Table 2 Analysis data of the XRPD pattern of the crystal form B of the compound (II)**

| **No.** | **2θ Angle (°)** | **D-spacing (Å)** | **Relative intensity (%)** | **No.** | **2θ Angle (°)** | **D-spacing (Å)** | **Relative intensity (%)** |
|---|---|---|---|---|---|---|---|
| 1 | 5.880 | 15.0174 | 100 | 14 | 21.286 | 4.1707 | 3.8 |
| 2 | 7.915 | 11.1608 | 6.5 | 15 | 21.956 | 4.0448 | 8.6 |
| 3 | 10.616 | 8.3267 | 2.2 | 16 | 23.867 | 3.7252 | 5.6 |
| 4 | 11.793 | 7.4978 | 23.2 | 17 | 24.248 | 3.6676 | 2.2 |
| 5 | 12.920 | 6.8464 | 8.3 | 18 | 26.668 | 3.3399 | 2.4 |
| 6 | 13.231 | 6.6859 | 1.3 | 19 | 27.517 | 3.2388 | 2.7 |
| 7 | 15.292 | 5.7892 | 1.5 | 20 | 28.646 | 3.1136 | 0.8 |
| 8 | 16.193 | 5.4692 | 2.3 | 21 | 29.054 | 3.0709 | 4.0 |
| 9 | 17.020 | 5.2051 | 1.4 | 22 | 29.689 | 3.0066 | 3.7 |
| 10 | 17.679 | 5.0126 | 8.3 | 23 | 31.271 | 2.8580 | 0.8 |
| 11 | 18.500 | 4.7919 | 4.3 | 24 | 34.635 | 2.5877 | 1.5 |
| 12 | 19.112 | 4.6398 | 1.3 | 25 | 34.911 | 2.5679 | 2.2 |
| 13 | 20.181 | 4.3964 | 1.8 | 26 | 35.818 | 2.5049 | 1.0 |

In some embodiments of the present disclosure, the above-mentioned crystal form B has a differential scanning calorimetry curve having an onset of an endothermic peak at 41.96±5°C, 87.83±5°C, and 165.00±5°C, and an onset of an exothermic peak at 176.55±5°C.

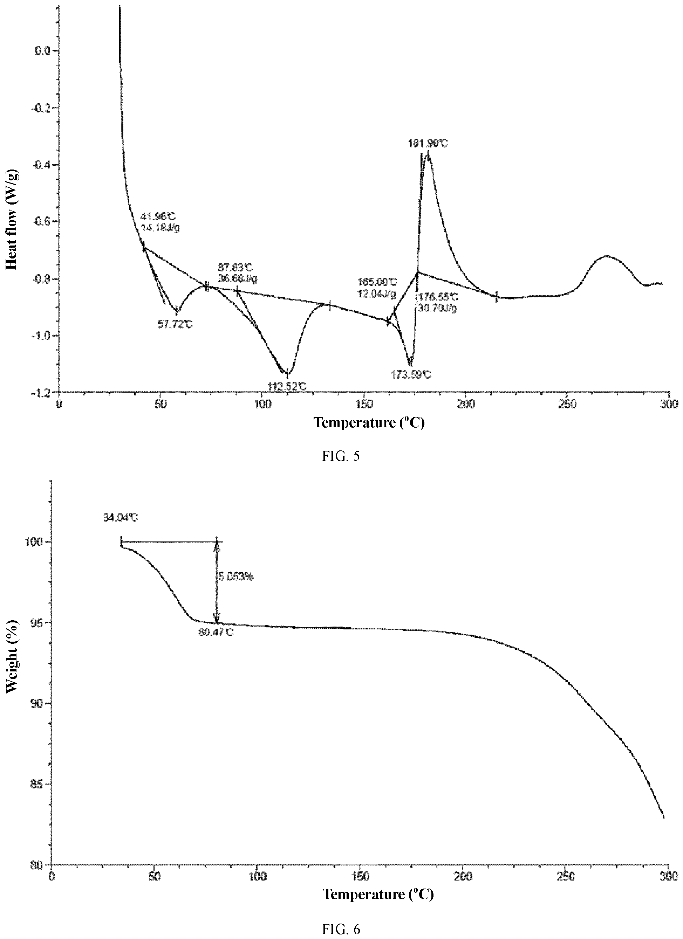

In some embodiments of the present disclosure, the DSC curve of the above-mentioned crystal form B is as shown in FIG. 5.

In some embodiments of the present disclosure, the above-mentioned crystal form B has a thermogravimetric analysis curve having a weight loss of up to 5.053% at 80.47±3°C.

In some embodiments of the present disclosure, the TGA curve of the above-mentioned crystal form B is as shown in FIG. 6.

The present disclosure also provides a crystal form C of the compound of formula (II), which has an X-ray powder diffraction pattern having characteristic diffraction peaks at 2θ angles of: 6.35±0.2°, 6.99±0.2°, and 13.02±0.2°.

In some embodiments of the present disclosure, the X-ray powder diffraction pattern of the above-mentioned crystal form C has characteristic diffraction peaks at 2θ angles of: 6.35±0.2°, 6.99±0.2°, 13.02±0.2°, 13.98±0.2°, 15.44± 0.2°, 15.95±0.2°, 19.08±0.2°, and 23.48±0.2° .

In some embodiments of the present disclosure, the X-ray powder diffraction pattern of the above-mentioned crystal form C has characteristic diffraction peaks at 2θ angles of: 6.35±0.2°, 6.99±0.2°, 13.02±0.2°, 13.98±0.2°, 15.44± 0.2°, 15.95±0.2°, 19.08±0.2°, 19.92±0.2°, 23.48±0.2°, 24.34±0.2°, 25.53±0.2°, and 28.13±0.2°.

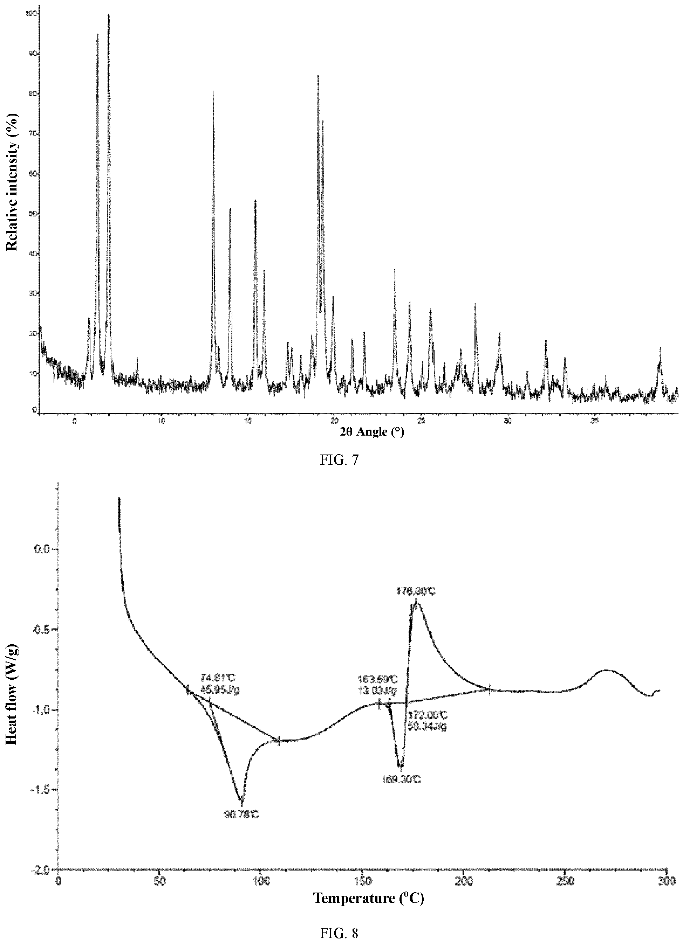

In some embodiments of the present disclosure, the XRPD pattern of the above-mentioned crystal form C is as shown in FIG. 7.

In some embodiments of the present disclosure, the analysis data of the XRPD pattern of the above-mentioned crystal form C is as shown in Table 3.

**Table 3 Analysis data of the XRPD pattern of the crystal form C of the compound of formula (II)**

| **No.** | **2θ Angle (°)** | **D-spacing (Å)** | **Relative intensity (%)** | **No.** | **2θ Angle (°)** | **D-spacing (Å)** | **Relative intensity (%)** |
|---|---|---|---|---|---|---|---|
| 1 | 5.829 | 15.1498 | 15.8 | 14 | 19.922 | 4.4531 | 21.9 |
| 2 | 6.350 | 13.9074 | 94.4 | 15 | 21.014 | 4.2240 | 13.8 |
| 3 | 6.985 | 12.6449 | 100 | 16 | 21.719 | 4.0884 | 16.1 |
| 4 | 13.022 | 6.7929 | 80.9 | 17 | 23.476 | 3.7864 | 32.9 |
| 5 | 13.300 | 6.6518 | 10.9 | 18 | 24.341 | 3.6536 | 24.1 |
| 6 | 13.983 | 6.3282 | 48.4 | 19 | 25.526 | 3.4867 | 22.4 |
| 7 | 15.443 | 5.7329 | 51.6 | 20 | 25.682 | 3.4659 | 13.3 |
| 8 | 15.954 | 5.5506 | 31.8 | 21 | 28.133 | 3.1693 | 22.9 |
| 9 | 17.296 | 5.1229 | 12.5 | 22 | 29.511 | 3.0243 | 16.6 |
| 10 | 17.534 | 5.0538 | 10.9 | 23 | 32.175 | 2.7797 | 14.0 |
| 11 | 18.684 | 4.7452 | 13.7 | 24 | 33.278 | 2.6901 | 10.2 |
| 12 | 19.078 | 4.6481 | 84.8 | 25 | 35.645 | 2.5167 | 6.3 |
| 13 | 19.311 | 4.5926 | 72.2 | 26 | 38.781 | 2.3201 | 13.8 |

In some embodiments of the present disclosure, the above-mentioned crystal form C has a differential scanning calorimetry curve having an onset of an endothermic peak at 74.81±5°C and 163.59±5°C, and an onset of an exothermic peak at 172.00±5°C.

In some embodiments of the present disclosure, the DSC curve of the above-mentioned crystal form C is as shown in FIG. 8.

In some embodiments of the present disclosure, the above-mentioned crystal form C has a thermogravimetric analysis curve having a weight loss of up to 5.462% at 115.95±3°C.

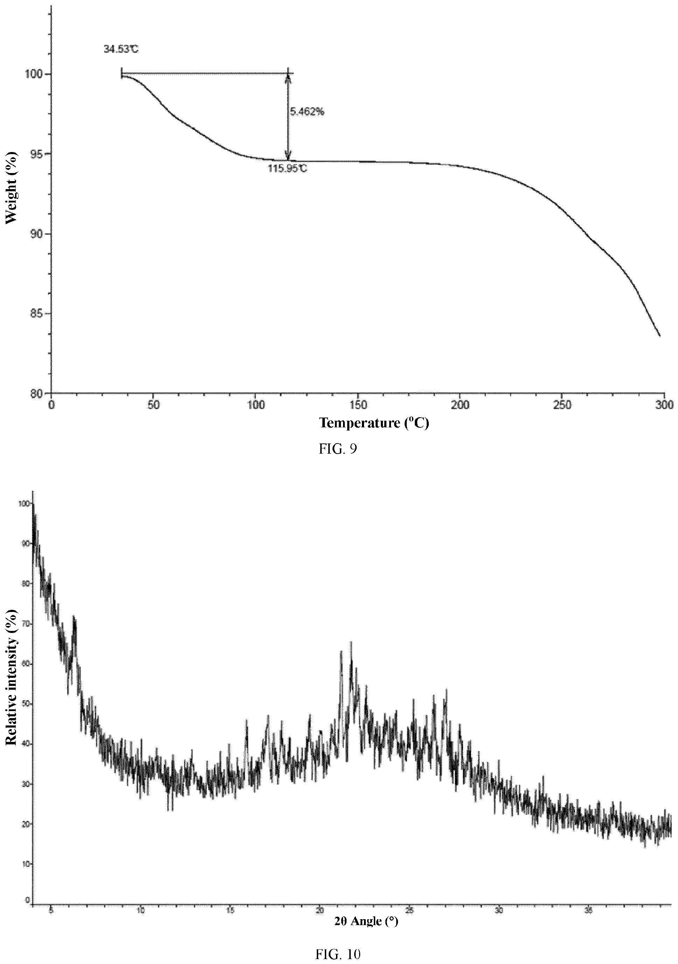

In some embodiments of the present disclosure, the TGA curve of the above-mentioned crystal form C is as shown in FIG. 9.

### Technical effect

The crystal forms of the compound of the present disclosure have good stability, good solubility, low hygroscopicity, and low susceptibility to light and heat, and have a good prospect of medicine.

### Definition and statement

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to the corresponding commodity or active ingredient thereof.

The intermediate compounds of the present disclosure can be prepared by a variety of synthetic methods well known to those skilled in the art, including specific embodiments listed below, embodiments formed by combining the specific embodiments listed below with other chemical synthetic methods, and equivalent alternative methods well known to those skilled in the art. The alternative embodiments include, but are not limited to, the examples of the present disclosure.

The chemical reactions in the specific embodiments disclosed herein are completed in a suitable solvent, which must be suitable for the chemical changes of the present disclosure and the reagents and materials required. In order to obtain the compound of the present disclosure, it is sometimes necessary for those skilled in the art to modify or select synthetic steps or reaction schemes based on the existing embodiments.

The present disclosure will be described in detail below through examples, which are not intended to limit the present disclosure in any way.

All solvents used in the present disclosure are commercially available and can be used without further purification.

The present disclosure uses the following abbreviations: r.t. stands for room temperature; RH stands for relative humidity; ΔW stands for weight increase by moisture absorption; MeOH stands for methanol; and HPLC stands for high performance liquid chromatography.

Compounds are named according to the conventional naming principle in the art or by ChemDraw^{®} software, and vendor directory names are used for commercially available compounds.

### 1. Instruments and analytical methods

### 1.1. X-ray powder diffractometer, XRPD

Instrument model: Bruker D8 advance X-ray diffractometer

Test conditions: The detailed XRPD parameters are as follows:
X-ray generator: Cu, kα, (λ=1.54056Å)
Tube voltage: 40 kV, tube current: 40 mA.
Scattering slit: 0.60 mm
Detector slit: 10.50 mm
Anti-scatter slit: 7.10 mm
Scan range: 3-40 degrees
Step size: 0.02 degrees
Step length: 0.12 seconds
Rotation speed of sample disk: 15 rpm

### 1.2. Differential Scanning Calorimeter, DSC

Instrument model: TA Q2000 Differential Scanning Calorimeter

Test conditions: The sample (0.5 to 1 mg) is weighed and placed in a DSC aluminum pot for testing. The method is carried out in a range of room temperature to 250°C at a heating rate of 10°C/min.

### 1.3. Thermal Gravimetric Analyzer, TGA

Instrument model: TA Q5000IR Thermogravimeter

Test conditions: The sample (2 to 5 mg) is weighed and placed in a TGA aluminum pot for testing. The method is carried out in a range of room temperature to 300°C at a heating rate of 10°C/min.

### 1.4. Dynamic Vapor Sorption, DVS method

Instrument model: SMS DVS Advantage dynamic vapor sorption apparatus

Test conditions: The sample (10 to 15 mg) is weighed and placed in a DVS sample pan for testing.

The detailed DVS parameters are as follows:
Temperature: 25 °C
Equilibration: dm/dt=0.01 %/min (The shortest time: 10 min, and the longest time: 180 min)
Drying: Dried for 120 min at 0% RH
RH (%) test gradient: 10%
Range of RH (%) test gradient: 0% - 90% - 0%

The classification of hygroscopicity evaluation is as follows:

| **Classification of hygroscopicity** | **ΔW(weight increase by moisture absorption*)** |
|---|---|
| Deliquescent | Absorbing enough water and forming a liquid |
| Very hygroscopic | ΔW% ≥ 15% |
| Hygroscopic | 15% > ΔW% ≥ 2% |
| Slightly hygroscopic | 2% > ΔW% ≥ 0.2% |
| Not or almost not hygroscopic | ΔW% < 0.2% |

| | |
|---|---|
| * weight increase by moisture absorption at 25 ± 1 °C and 80 ± 2% RH. | |

### BRIEF DESCRIPTION OF THE DRAWINGS

FIG. 1 is the XRPD pattern of the crystal form A of the compound of formula (II).
FIG. 2 is the DSC curve of the crystal form A of the compound of formula (II).
FIG. 3 is the TGA curve of the crystal form A of the compound of formula (II).
FIG. 4 is the XRPD pattern of the crystal form B of the compound of formula (II).
FIG. 5 is the DSC curve of the crystal form B of the compound of formula (II).
FIG. 6 is the TGA curve of the crystal form B of the compound of formula (II).
FIG. 7 is the XRPD pattern of the crystal form C of the compound of formula (II).
FIG. 8 is the DSC curve of the crystal form C of the compound of formula (II).
FIG. 9 is the TGA curve of the crystal form C of the compound of formula (II).
FIG. 10 is the XRPD pattern of the sulfate of the compound of formula (I).
FIG. 11 is the XRPD pattern of the phosphate of the compound of formula (I).
FIG. 12 is the XRPD pattern of the crystal form D of the maleate of the compound of formula (I).
FIG. 13 is the DSC curve of the crystal form D of the maleate of the compound of formula (I).
FIG. 14 is the TGA curve of the crystal form D of the maleate of the compound of formula (I).
FIG. 15 is the XRPD pattern of the L-tartrate of the compound of formula (I).
FIG. 16 is the XRPD pattern of the formate of the compound of formula (I).
FIG. 17 is the DVS curve of the crystal form A of the compound of formula (II).
FIG. 18 is the DVS curve of the crystal form C of the compound of formula (II).

### DETAILED DESCRIPTION OF THE INVENTION

In order to better understand the content of the present disclosure, the present disclosure is further illustrated below in conjunction with specific examples, but the specific examples are not intended to limit the content of the present disclosure.

### Example 1: Preparation of crystal form A of the compound of formula (II)

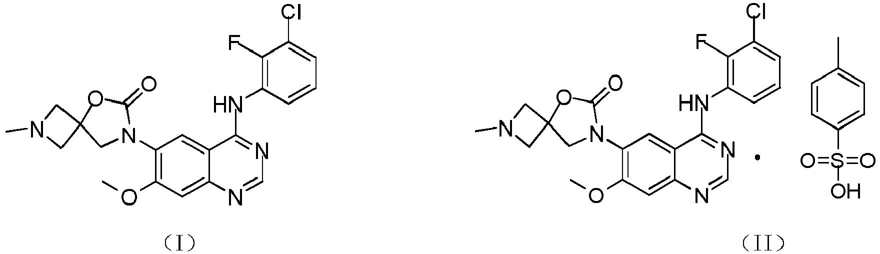

### Method 1:

To a 100 ml three-necked flask was added 15 ml of anhydrous methanol, and the compound of formula (I) (3.0 g, 6.76 mmol, 1.0 equiv) was added to the three-necked flask at room temperature. The mixture was heated to 70°C. P-toluenesulfonic acid (1.29 g, 6.76 mmol, 1.0 equiv) was dissolved in 2 ml of methanol, and the solution was slowly added dropwise to the above reaction solution. After the addition was completed, the solution was clear. The solution was stirred while maintaining the temperature for 1 hour, and then cooled down to 20-30°C. The mixture under N₂ was filtered with suction under reduced pressure, and the filter cake was dried to constant weight in a vacuum drying oven at 40-50°C to give the crystal form A of the compound of formula (II). ¹H NMR (400MHz, deuterated methanol) δ = 8.49 (s, 1H), 8.44 (s, 1H), 7.72 - 7.65 (m, 2H), 7.58 - 7.52 (m, 1H), 7.45 - 7.37 (m, 1H), 7.33 - 7.28 (m, 1H), 7.27 - 7.14 (m, 3H), 4.70 - 4.54 (m, 4H), 4.41 (s, 2H), 4.08 (s, 3H), 3.09 (s, 3H), 2.36 (s, 3H); LCMS (ESI) m/z: 444.1 [M+1].

The XRPD pattern of the crystal form A of the compound of formula (II) is as shown in FIG. 1, the DSC curve is as shown in FIG. 2, and the TGA curve is as shown in FIG. 3.

### Method 2:

30 mg of the crystal form A of the compound of formula (II) was weighed and added to a 1.5 ml glass vial, and an appropriate amount of n-heptane was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form A of the compound of formula (II).

### Method 3:

30 mg of the crystal form A of the compound of formula (II) was weighed and added to a 1.5 ml glass vial, and an appropriate amount of a mixed solvent of methanol and H₂O (methanol:H₂O=10:1 (V:V)) was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form A of the compound of formula (II).

### Example 2: Preparation of the crystal form B of the compound of formula (II)

### Method 1:

30 mg of the crystal form A of the compound of formula (II) was weighed and added to a 1.5 ml glass vial, and an appropriate amount of ethanol was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form B of the compound of formula (II). ¹H NMR (400 MHz, deuterated DMSO) δ = 10.34 - 9.65 (m, 1H), 8.54 - 8.45 (m, 2H), 7.56 - 7.43 (m, 4H), 7.30 (s, 1H), 7.24 - 7.21 (m, 1H), 7.14 - 7.04 (m, 2H), 4.69 - 4.35 (m, 4H), 4.32 - 4.19 (m, 2H), 4.05 - 3.95 (m, 3H), 2.30 - 2.27 (m, 3H).

The XRPD pattern of the crystal form B is as shown in FIG. 4, the DSC curve is as shown in FIG. 5, and the TGA curve is as shown in FIG. 6.

### Method 2:

30 mg of the crystal form A of the compound of formula (II) was weighed and added to a 1.5 ml glass vial, and an appropriate amount of acetone was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form B of the compound of formula (II).

### Method 3:

30 mg of the crystal form A of the compound of formula (II) was weighed and added to a 1.5 ml glass vial, and an appropriate amount of tetrahydrofuran was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form B of the compound of formula (II).

### Method 4:

30 mg of the crystal form A of the compound of formula (II) was weighed and added to a 1.5 ml glass vial, and an appropriate amount of acetonitrile was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form B of the compound of formula (II).

### Method 5:

30 mg of the crystal form A of the compound of formula (II) was weighed and added to a 1.5 ml glass vial, and an appropriate amount of ethyl acetate was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form B of the compound of formula (II).

### Method 6:

30 mg of the crystal form A of the compound of formula (II) was weighed and added to a 1.5 ml glass vial, and an appropriate amount of methyl isobutyl ketone was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form B of the compound of formula (II).

### Example 3: Preparation of the crystal form C of the compound of formula (II)

### Method 1:

30 mg of the crystal form A of the compound of formula (II) was weighed and added to a 1.5 ml glass vial, and an appropriate amount of ethanol and H₂O (ethanol:H₂O=10:1 (V:V)) was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form C of the compound of formula (II). ¹H NMR (400 MHz, deuterated DMSO) δ = 10.03 - 9.92 (m, 1H), 8.57 - 8.42 (m, 2H), 7.56 - 7.44 (m, 4H), 7.40 - 7.20 (m, 2H), 7.15 - 7.03 (m, 2H), 4.66 - 4.35 (m, 4H), 4.33 - 4.21 (m, 2H), 4.05 - 3.96 (m, 3H), 2.30 - 2.24 (m, 3H)

The XRPD pattern of the crystal form C of the compound of formula (II) is as shown in FIG. 7, the DSC curve is as shown in FIG. 8, and the TGA curve is as shown in FIG. 9.

### Method 2:

30 mg of the crystal form A of the compound of formula (II) was weighed and added to a 1.5 ml glass vial, and an appropriate amount of isopropanol and H₂O (isopropanol:H₂O=10:1 (V:V)) was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form C of the compound of formula (II).

### Method 3:

30 mg of the crystal form A of the compound of formula (II) was weighed and added to a 1.5 ml glass vial, and an appropriate amount of acetone and H₂O (acetone:H₂O=10:1 (V:V)) was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form C of the compound of formula (II).

### Method 4:

30 mg of the crystal form A of the compound of formula (II) was weighed and added to a 1.5 ml glass vial, and an appropriate amount of THF and H₂O (THF:H₂O=10:1 (V:V)) was added so that the mixture formed a suspension. After adding a stirring bar, the above suspension sample was placed on a magnetic stirrer with heating (40°C) for testing (protected from light), stirred at 40°C for 2 days, and then centrifuged. The residual solid sample was dried in a vacuum drying oven (30°C) overnight (10-16 hours) to give the crystal form C of the compound of formula (II).

### Example 4: Preparation of the compound of formula (I) hydrochloride

About 30 mg of the compound of formula (I) was weighed and added to a 1.5 mL vial, and 1.0 mL of ethanol was added. The sample was placed on a magnetic stirrer (40°C) and stirred, and then hydrochloric acid (58.34 µL) was added. It was found that the solution became clear. The solution was stirred while maintaining the temperature for 48 hours. The solution was still clear. The clear solution was subjected to a volatilization test at 40°C. The sample became gelatinous or oily. Ethyl acetate was added and the solution was stirred for crystallization. No solid was found to precipitate.

### Example 5: Preparation of the sulfate of the compound of formula (I)

About 30 mg of the compound of formula (I) was weighed and added to a 1.5 mL vial, and 1.0 mL of ethanol was added. The sample was placed on a magnetic stirrer (40°C) and stirred, and then sulfuric acid (38.60 µL) was added. The solution was stirred while maintaining the temperature for 48 hours. A large amount of solids precipitated from the solution. The solution was centrifuged to give the sulfate of the compound of formula (I).

The sulfate of the compound of formula (I) is amorphous, and the XRPD pattern is as shown in FIG. 10.

### Example 6: Preparation of the phosphate of the compound of formula (I)

About 30 mg of the compound of formula (I) was weighed and added to a 1.5 mL vial, and 1.0 mL of ethanol was added. The sample was placed on a magnetic stirrer (40°C) and stirred, and then phosphoric acid (48.41 µL) was added. The solution was stirred while maintaining the temperature for 48 hours. A large amount of solids precipitated from the solution. The solution was centrifuged, and the filter cake was dried to constant weight in a vacuum drying oven at 40-50°C to give the phosphate of the compound of formula (I).

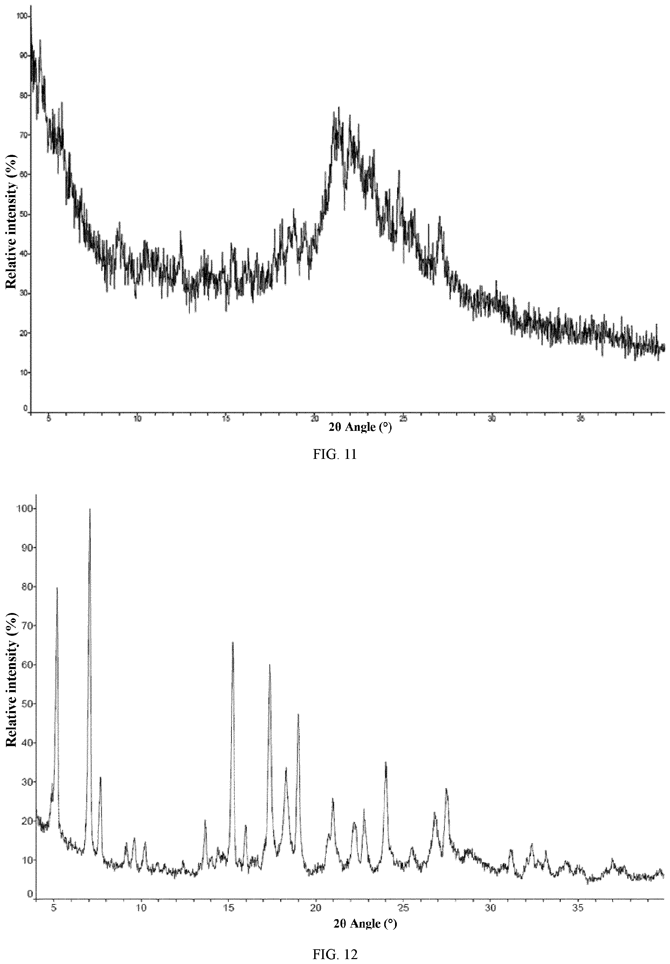

The phosphate of the compound of formula (I) is amorphous, and the XRPD pattern is as shown in FIG. 11.

### Example 7: Preparation of the methanesulfonate of the compound of formula (I)

About 30 mg of the compound of formula (I) was weighed and added to a 1.5 mL vial, and 1.0 mL of ethanol was added. The sample was placed on a magnetic stirrer (40°C) and stirred, and then methanesulfonic acid (46.04 µL) was added. The solution was stirred while maintaining the temperature for 48 hours. A large amount of solids precipitated from the solution. The solution was centrifuged to give the methanesulfonate of the compound of formula (I).

### Example 8: Preparation of the crystal form D of the maleate of the compound of formula (I)

To a 100 ml three-necked flask was added 20 ml of anhydrous methanol, and the compound of formula (I) (3.0 g, 6.57 mmol, 1.0 equiv) was added to the three-necked flask at room temperature. The mixture was heated to 70°C, and maleic acid (1.91 g, 16.43 mmol, 2.5 equiv) dissolved in 10 ml of methanol was slowly added dropwise to the above reaction solution. After the addition was completed, the solution was clear. After 5 minutes, white solids precipitated from the solution. The solution was stirred while maintaining the temperature for 2 hours, and then cooled down to 20-30°C. The mixture under N₂ was filtered with suction under reduced pressure, and the filter cake was dried to constant weight in a vacuum drying oven at 40-50°C to give the crystal form D of the maleate of the compound of formula (I). ¹H NMR (400MHz, deuterated methanol) δ = 8.60 (s, 1H), 8.48 (s, 1H), 7.63 - 7.53 (m, 1H), 7.53 - 7.44 (m, 1H), 7.37 (s, 1H), 7.28 (dt, J=1.2, 8.1 Hz, 1H), 6.29 (s, 4H), 4.75 - 4.60 (m, 4H), 4.40 (s, 2H), 4.11 (s, 3H), 3.10 (s, 3H). LCMS (ESI) m/z: 444.1 [M+1].

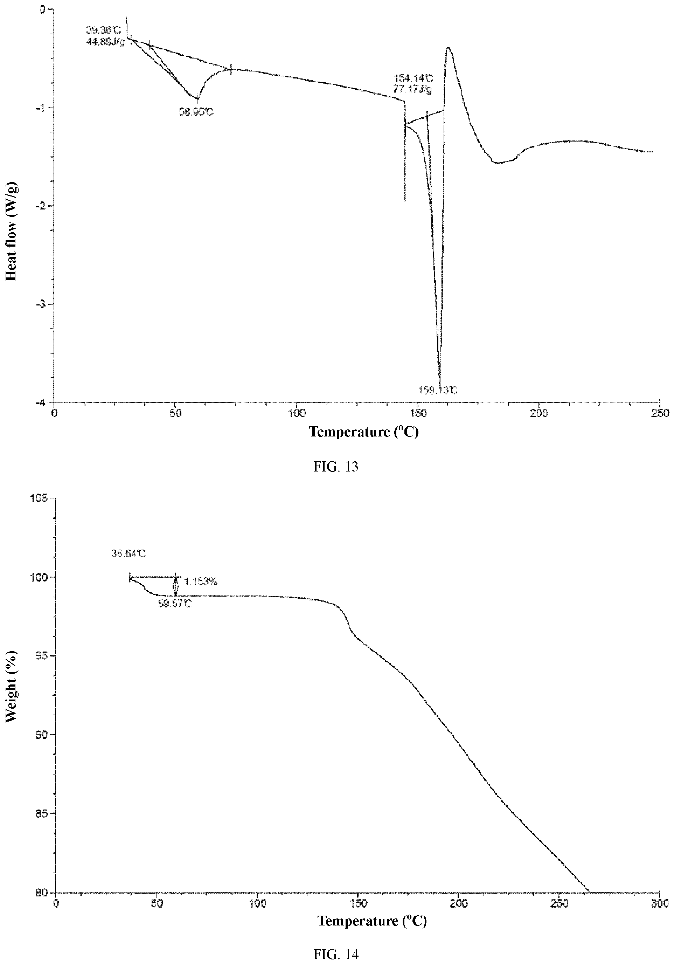

The XRPD pattern of the crystal form D of the maleate of the compound of formula (I) is as shown in FIG. 12, the DSC curve is as shown in FIG. 13, and the TGA curve is as shown in FIG. 14.

### Example 9: Preparation of the L-tartrate of the compound of formula (I)

About 30 mg of the compound of formula (I) was weighed and added to a 1.5 mL vial, and 1.0 mL of ethanol was added. The sample was placed on a magnetic stirrer (40°C) and stirred, and then L-tartaric acid (11.2mg) was added. The solution was stirred while maintaining the temperature for 48 hours. A large amount of solids precipitated from the solution. The solution was centrifuged, and the filter cake was dried to constant weight in a vacuum drying oven at 40-50°C to give the L-tartrate of the compound of formula (I).

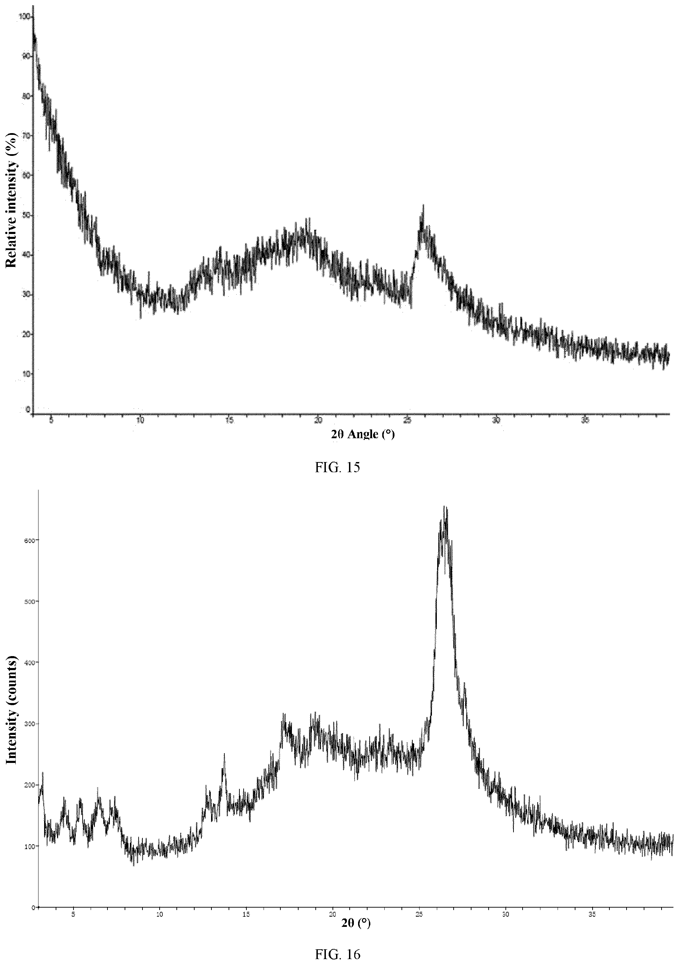

The tartrate of the compound of formula (I) is amorphous, and the XRPD pattern is as shown in FIG. 15.

### Example 10: Preparation of the formate of the compound of formula (I)

About 300 mg of the compound of formula (I) was weighed and added to a 100 mL round bottom flask, and 10 mL of acetonitrile and 10 ml of purified water were added. 31 mg of formic acid was added to the above reaction flask. The mixture was lyophilized to give the formate of the compound of formula (I). ¹HNMR (400MHz, deuterated methanol) δ = 8.45 (s, 1H), 8.36 (s, 1H), 8.28 (s, 1H), 7.60 - 7.54 (m, 1H), 7.40 (ddd, J=1.6, 6.7, 8.2 Hz, 1H), 7.31 (s, 1H), 7.22 (dt, J=1.5, 8.1 Hz, 1H), 4.30 (s, 2H), 4.15 - 4.08 (m, 4H), 4.05 (s, 3H), 2.74 (s, 3H).

The formate of the compound of formula (I) is amorphous, and the XRPD pattern is as shown in FIG. 16.

### Example 11: Study on the hygroscopicity of the crystal form A and the crystal form C of the compound of formula (II)

Materials of the assay:
Dynamic vapor sorption apparatus (SMS DVS Advantage)
Method of the assay:
10-15 mg of the crystal form A and the crystal form C of the compound of formula (II) were weighed and placed into a DVS sample pan for testing.

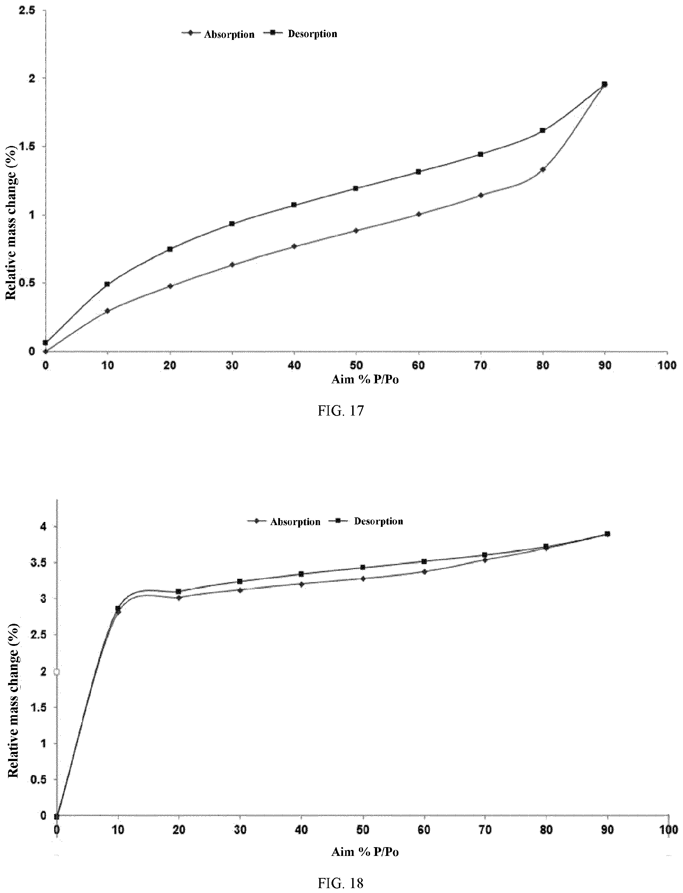

Results of the assay:
The DVS curve of the crystal form A of the compound of formula (II) is as shown in FIG. 17, wherein ΔW is equal to 1.329% at 25°C and 80% RH
The DVS curve of the crystal form C of the compound of formula (II) is as shown in FIG. 18, wherein ΔW is equal to 3.697% at 25°C and 80% RH

### Conclusion of the assay:

The crystal form A of the compound of formula (II) is slightly hygroscopic, and the crystal form C of the compound of formula (II) is hygroscopic.

### Example 12: Water activity assay of the crystal form A of the compound of formula (II)

### Purpose of the assay:

The stability of the crystal form in water with different activities was determined.

### Method of the assay:

About 30 mg of the crystal form A of the compound of formula (II) was weighed and placed in a 1.5 mL liquid phase vial. A magnetic bar was added, and an appropriate amount of water activity agent in a system of methanol and water was pipetted into the liquid phase vial. The suspension sample was mixed well and then placed on a magnetic stirrer (25°C, 700rpm). After stirring for two days, the wet sample was determined by XRPD.

### Conclusion of the assay:

When the crystal form A of the compound of formula (II) is in water with an activity of 0.1 and 0.3 at 25°C, the crystal form is not changed. When the activity of water is greater than 0.3, the crystal form A is changed to the crystal form C.

### Example 13: Solubility assay of the crystal form A of the compound of formula (II) in media with different pHs

9 parts of 4 mg of the crystal form A of the compound of formula (II) were weighed and added to a 1.5 mL sample bottle, respectively, and then 1 mL of different media (0.1 mol/L HCl, 0.01 mol/L HCl, pH3.8 buffer solution, pH4.5 buffer solution, pH5.5 buffer solution, pH6.0 buffer solution, pH6.8 buffer solution, pH7.4 buffer solution, and water) were added, respectively. Based on the dissolution situation, the raw material compound was continuously added until a saturated solution was formed. The magnetic bar was added to the above suspension, and the suspension was placed on a magnetic stirrer (37°C, protected from light) and stirred. After stirring for 24 hours, the sample was taken and centrifuged. The upper layer of the sample was filtered with a filter membrane, and the pH value of the filtrate was determined. The saturated solubility of the compound was determined by HPLC. The determination results were shown in Table 4.

**Table 4 Results of solubility determination of the crystal form A of the compound of formula (II) in buffer solutions with different pHs**

| **Medium** | **Solubility (2 hours, mg/mL)** | **Solubility (24 hours, mg/mL)** | **Evaluation of dissolution property** | **Final pH** |
|---|---|---|---|---|
| 0.1 mol/L HCl | >10 | >10 | High solubility | --- |
| 0.01 mol/L HCl | 7.444 | 7.941 | | 3.59 |
| pH 3.8 buffer solution | 3.749 | 3.645 | | 3.988 |
| pH 4.5 buffer solution | 3.824 | 3.141 | | 4.531 |
| pH 5.5 buffer solution | 3.571 | 1.299 | | 5.499 |
| pH 6.0 buffer solution | 3.785 | 3.638 | | 5.900 |
| water | 2.720 | 1.142 | | 5.296 |
| pH 6.8 buffer solution | 0.354 | 0.073 | Low solubility | 6.588 |
| pH 7.4 buffer solution | 0.099 | 0.022 | | 7.096 |

### Conclusion: The crystal form A of the compound of formula (II) shows high solubility in buffer solutions below pH 6.8 and purified water.

### Example 14: Solubility assay of the crystal form C of the compound of formula (II) in media with different pHs

9 parts of 4 mg of the crystal form C of the compound of formula (II) were weighed and added to a 1.5 mL sample bottle, respectively, and then 1 mL of different media (0.1 mol/L HCl, 0.01 mol/L HCl, pH3.8 buffer solution, pH4.5 buffer solution, pH5.5 buffer solution, pH6.0 buffer solution, pH6.8 buffer solution, pH7.4 buffer solution, and water) were added, respectively. Based on the dissolution situation, the raw material compound was continuously added until a saturated solution was formed. The magnetic bar was added to the above suspension, and the suspension was placed on a magnetic stirrer (37°C, protected from light) and stirred. After stirring for 24 hours, the sample was taken and centrifuged. The upper layer of the sample was filtered with a filter membrane, and the pH value of the filtrate was determined. The saturated solubility of the compound was determined by HPLC. The determination results were shown in Table 5.

**Table 5 Results of solubility determination of the crystal form C of the compound of formula (II) in buffer solutions with different pHs**

| **Medium** | **Solubility (2 hours, mg/mL)** | **Solubility (24 hours, mg/mL)** | **Evaluation of dissolution property** | **Final pH** |
|---|---|---|---|---|
| 0.1 mol/L HCl | >10 | >10 | High solubility | - |
| 0.01 mol/L HCl | 5.666 | 2.863 | | 2.863 |
| pH 3.8 buffer solution | 1.612 | 1.681 | | 3.928 |
| pH 4.5 buffer solution | 1.167 | 1.186 | | 4.560 |
| pH 5.5 buffer solution | 0.916 | 0.863 | | 5.528 |
| pH 6.0 buffer solution | 0.699 | 0.328 | | 5.708 |
| water | 0.720 | 0.646 | | 5.444 |
| pH 6.8 buffer solution | 0.260 | 0.071 | Low solubility | 6.625 |
| pH 7.4 buffer solution | 0.081 | 0.040 | | 7.142 |

Conclusion: The crystal form C of the compound of formula (II) shows high solubility in buffer solutions below pH 6.8 and purified water.

### Example 15: Stability assay of the crystal form A and the crystal form C of the compound (II)

### Purpose of the assay:

The stability of crystal form of the crystal form A and the crystal form C of the compound of formula (II) under the conditions of illumination, high humidity (92.5% RH), high temperature (60 °C) and acceleration (60°C/75%RH) was investigated.

### Instrument for the assay:

Illumination test chamber, model: SHH-100GD-2, conditions: 5000±500 lux (visible light) and 90 mw/cm² (ultraviolet).

Electrothermal blowing dry box, model: GZX-9140MBE, conditions: 60°C.

Constant temperature and humidity chamber, model: LDS-800Y, conditions: 40°C/75%RH

Constant temperature and humidity chamber, model: SHH-250SD, conditions: 25°C, 92.5%RH

### Method of the assay:

An appropriate amount of the crystal form A of the compound of formula (II) was weighed and placed in a dry and clean weighing bottle. The sample was spread into a thin layer, and fully exposed to assay conditions (60°C, 92.5% RH, illumination) and acceleration conditions (40°C /75%RH). After 17 days, the compound was taken out and determined for related substances and crystal form.

An appropriate amount of the crystal form C of the compound of formula (II) was weighed and placed in a dry and clean weighing bottle. The sample was spread into a thin layer, and fully exposed to assay conditions (60°C, 92.5% RH, illumination) and acceleration conditions (40°C /75%RH). After 10 days, the compound was taken out and determined for related substances and crystal form.

### Results of the assay:

The changes of related substances of the crystal form A of the compound of formula (II) under the conditions of illumination, high temperature and acceleration were shown in Table 6.

**Table 6**

| **Relative retention time** | 0.24 | 0.67 | 0.88 | 0.92 | 1.24 | 1.30 | 1.56 | 1.76 | **Total impurity** |
|---|---|---|---|---|---|---|---|---|---|
| **Initial sample** | 0.03 | 0.05 | 0.03 | | 0.15 | 0.18 | 0.04 | | 0.53 |
| **Illumination-17 days** | 0.03 | 0.07 | 0.04 | 0.05 | 0.13 | 0.17 | 0.04 | | 0.46 |
| **60°C-17 days** | 0.03 | 0.91 | 0.05 | 0.03 | 0.15 | 0.16 | 0.04 | 0.07 | 1.44 |
| **92.5%RH-17 days** | 0.03 | 0.05 | 0.04 | 0.04 | 0.14 | 0.18 | 0.04 | 0.02 | 0.54 |
| **40°C-75% RH -17 days** | 0.03 | 0.16 | 0.04 | 0.04 | 0.15 | 0.18 | 0.04 | 0.04 | 0.66 |

The changes of related substances of the crystal form C of the compound of formula (II) under the conditions of illumination, high temperature and acceleration were shown in Table 7.

**Table 7**

| | | | | | | | | | | | | |
|---|---|---|---|---|---|---|---|---|---|---|---|---|
| **Relative retention time** | 0.24 | 0.69 | 0.81 | 0.88 | 0.92 | 1.15 | 1.17 | 1.24 | 1.30 | 1.81 | 1.89 | **Total impurity** |
| **Initial sample** | 0.02 | 0.04 | - | 0.02 | 0.02 | - | | 0.12 | 0.17 | | | 0.42 |
| **Illumination-10 days** | 0.02 | 0.04 | 0.04 | | 0.05 | 0.02 | 0.03 | 0.11 | 0.17 | | | 0.40 |
| **60°C-10 days** | 0.02 | 0.26 | | 0.03 | | | | 0.11 | 0.17 | 0.05 | 0.05 | 0.69 |
| **92.5%RH-10 days** | 0.02 | 0.04 | | 0.02 | | | | 0.12 | 0.17 | | | 0.37 |
| **40°C-75% RH -10 days** | 0.03 | 0.06 | | 0.04 | 0.02 | | | 0.14 | 0.17 | 0.03 | | 0.53 |

### Conclusions of the assay:

The crystal form of the crystal form A of the compound of formula (II) is not changed under the conditions of illumination, high temperature and acceleration, but the crystal form is changed under the condition of high humidity (92.5%RH).

The crystal form A of the compound of formula (II) produces a degradation impurity only under the condition of high temperature, and its stability is better than that of the crystal form D of the maleate of the compound of formula (I).

The crystal form of the crystal form C of the compound of formula (II) is not changed under the conditions of illumination, high temperature, high humidity and acceleration, and the crystal form is relatively stable.

The crystal form C of the compound of formula (II) produces a degradation impurity only under the condition of high temperature, and its stability is better than that of the crystal form D of the maleate of the compound of formula (I).

### Example 16: Stability assay of the crystal form D of the maleate of the compound of formula (I)

### Purpose of the assay:

The stability of crystal form of the crystal form D of the maleate of the compound of formula (I) under the conditions of illumination (5000±500 lux(visible light) and 90□/cm² (ultraviolet)), high humidity (92.5%RH), high temperature (60°C) and acceleration (40°C/75%RH) was investigated.

### Instrument for the assay:

Illumination test chamber, model: SHH-100GD-2, conditions: 5000±500 lux (visible light) and 90mw/cm² (ultraviolet).

Electrothermal blowing dry box, model: GZX-9140MBE, conditions: 60°C.

Constant temperature and humidity chamber, model: LDS-800Y, conditions: 40°C/75%RH.

Constant temperature and humidity chamber, model: SHH-250SD, conditions: 25°C, 92.5%RH.

### Method of the assay:

An appropriate amount of the crystal form D of the maleate of the compound of formula (I) was weighed and placed in a dry and clean weighing bottle. The sample was spread into a thin layer, and fully exposed to assay conditions (60°C, 92.5% RH, illumination) and acceleration conditions (40°C /75%RH). After 10 days, the compound was taken out and determined for related substances and crystal form.

### Conclusions of the assay:

The crystal form of the crystal form D of the maleate of the compound of formula (I) is not changed under the conditions of illumination and high temperature, but the crystal form is changed under the conditions of high humidity (92.5%RH) and acceleration (40°C/75%RH).

The changes of related substances of the crystal form D of the maleate of the compound of formula (I) under the conditions of illumination, high temperature, and acceleration were shown in Table 8.

**Table 8**

| **Relative retention time** | 0.98 | 0.98 | 1.04 | 1.06 | 1.09 | 1.13 | 1.23 | 1.41 | 1.44 | 1.48 | 1.49 | **Total impurity** |
|---|---|---|---|---|---|---|---|---|---|---|---|---|
| **Initial sample** | | | 0.12 | 0.29 | 0.06 | | | | 0.89 | | 0.08 | 1.44 |
| **Illumination** | | 0.07 | 0.11 | 0.29 | | | | | 0.69 | | 0.07 | 1.23 |
| **60°C-10 days** | 0.09 | 0.15 | 0.35 | 0.34 | 0.07 | 0.17 | 0.1 | 0.08 | 0.62 | 0.05 | 0.06 | 2.08 |
| **92.5%RH-10 days** | | 0.07 | 0.28 | 0.29 | 0.06 | | | | 0.88 | | 0.08 | 1.65 |
| **40°C-75%RH-10 days** | | | 0.37 | 0.29 | 0.07 | | 0.1 | | 0.64 | 0.14 | 0.06 | 1.67 |

It shows that the compound of formula (I) will produce relatively more degradation impurities under the conditions of high temperature and acceleration, and its stability is lower than that of the compound of formula (II).

## Claims

1. A crystal form A of the compound of formula (II), which has an X-ray powder diffraction pattern having characteristic diffraction peaks at 2θ angles of: 6.04±0.2°, 12.07±0.2°, and 13.32±0.2°,

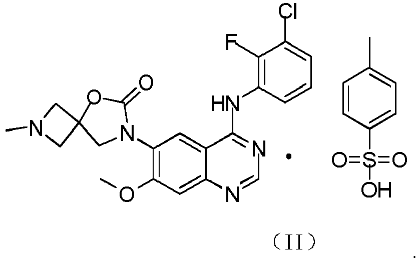

2. The crystal form A according to claim 1, wherein the X-ray powder diffraction pattern has characteristic diffraction peaks at 2θ angles of: 6.04±0.2°, 12.07±0.2°, 13.32±0.2°, 16.06±0.2°, 19.01±0.2°, 20.14±0.2°, 25.07±0.2°, and 30.44±0.2°.

3. The crystal form A according to claim 2, wherein the X-ray powder diffraction pattern has characteristic diffraction peaks at 2θ angles of: 6.04±0.2°, 8.26±0.2°, 10.79±0.2°, 11.27±0.2°, 12.07±0.2°, 13.32±0.2°, 16.06±0.2°, 19.01±0.2°, 20.14±0.2°, 23.02±0.2°, 25.07±0.2°, and 30.44±0.2°.

4. The crystal form A according to claim 3, wherein the XRPD pattern is as shown in FIG. 1.

5. The crystal form A according to any one of claims 1-4, which has a differential scanning calorimetry curve having an onset of an endothermic peak at 200.99±5°C and an onset of an exothermic peak at 212.33±5°C.

6. The crystal form A according to claim 5, wherein the DSC curve is as shown in FIG. 2.

7. The crystal form A according to any one of claims 1-4, which has a thermogravimetric analysis curve having a weight loss of up to 0.2289% at 120.00±3°C.

8. The crystal form A according to claim 7, wherein the TGA curve is as shown in FIG. 3.

9. A crystal form B of the compound of formula (II), which has an X-ray powder diffraction pattern having characteristic diffraction peaks at 2θ angles of: 5.88±0.2°, 11.79±0.2°, and 21.96±0.2°, .

10. The crystal form B according to claim 9, wherein the X-ray powder diffraction pattern has characteristic diffraction peaks at 2θ angles of: 5.88±0.2°, 7.92±0.2°, 11.79±0.2°, 12.92±0.2°, 17.68±0.2°, 18.50±0.2°, 21.96±0.2°, and 23.87±0.2°.

11. The crystal form B according to claim 10, wherein the X-ray powder diffraction pattern has characteristic diffraction peaks at 2θ angles of: 5.88±0.2°, 7.92±0.2°, 11.79±0.2°, 12.92±0.2°, 17.68±0.2°, 18.50±0.2°, 21.29±0.2°, 21.96±0.2°, 23.87±0.2°, 27.52±0.2°, 29.05±0.2°, and 29.69±0.2°.

12. The crystal form B according to claim 11, wherein the XRPD pattern is as shown in FIG. 4.

13. The crystal form B according to any one of claims 9-12, which has a differential scanning calorimetry curve having an onset of an endothermic peak at 41.96±5°C, 87.83±5°C, and 165.00±5°C, and an onset of an exothermic peak at 176.55±5°C.

14. The crystal form B according to claim 13, wherein the DSC curve is as shown in FIG. 5.

15. The crystal form B according to any one of claims 9-12, which has a thermogravimetric analysis curve having a weight loss of up to 5.053% at 80.47±3°C.

16. The crystal form B according to claim 15, wherein the TGA curve is as shown in FIG. 6.

17. A crystal form C of the compound of formula (II), which has an X-ray powder diffraction pattern having characteristic diffraction peaks at 2θ angles of: 6.35±0.2°, 6.99±0.2°, and 13.02±0.2°,

18. The crystal form C according to claim 17, wherein the X-ray powder diffraction pattern has characteristic diffraction peaks at 2θ angles of: 6.35±0.2°, 6.99±0.2°, 13.02±0.2°, 13.98±0.2°, 15.44±0.2°, 15.95±0.2°, 19.08±0.2°, and 23.48±0.2°.

19. The crystal form C according to claim 18, wherein the X-ray powder diffraction pattern has characteristic diffraction peaks at 2θ angles of: 6.35±0.2°, 6.99±0.2°, 13.02±0.2°, 13.98±0.2°, 15.44±0.2°, 15.95±0.2°, 19.08±0.2°, 19.92±0.2°, 23.48±0.2°, 24.34±0.2°, 25.53±0.2°, and 28.13±0.2°.

20. The crystal form C according to claim 19, wherein the XRPD pattern is as shown in FIG. 7.

21. The crystal form C according to any one of claims 17-20, which has a differential scanning calorimetry curve having an onset of an endothermic peak at 74.81±5°C and 163.59±5°C, and an onset of an exothermic peak at 172.00±5°C.

22. The crystal form C according to claim 21, wherein the DSC curve is as shown in FIG. 8.

23. The crystal form C according to any one of claims 17-20, which has a thermogravimetric analysis curve having a weight loss of up to 5.462% at 115.95±3°C.

24. The crystal form C according to claim 23, wherein the TGA curve is as shown in FIG. 9.
